# DEMANDE DE BREVET EUROPEEN

(11) **EP 3 682 808 A1**
(43) Date de publication de la demande: **22.07.2020**
(21) Numéro de dépôt: 19020031.1
(22) Date de dépôt: 17.01.2019
(51) Int. Cl.: A61B 6/03, A61B 6/12, A61B 6/00, G06F 3/01, G02B 27/01, A61B 17/34, G06T 19/00, A61B 34/20, A61B 34/10

(54) **DISPOSITIF MÉDICAL EN RADIOLOGIE INTERVENTIONNELLE DE GUIDAGE EN TEMPS RÉEL D'UNE AIGUILLE MÉDICALE D'OPÉRATION DANS UN VOLUME**

(71) Demandeur: Université Paris-Est Créteil Val de Marne, 94010 Creteil Cedex (FR); Assistance Publique Hopitaux des Paris (APHP), 75184 Paris (FR); Centre National de la Recherche Scientifique, 75016 Paris Cedex 16 (FR); Chambre de Commerce et d'Industrie de Region Paris Ile de France, 75008 Paris (FR)
(72) Inventeur: TACHER, Vania, 75012 Paris (FR); PICO, Christopher, 77220 Tournan en Brie (FR); KOBEITER, Hicham, 75013 Paris (FR); GRANDPIERRE, Thierry, 93700 Drancy (FR); SACCENTI, Laetitia, 75011 Paris (FR)
(74) Mandataire: Balmary, Simon

(57) **Abrégé**

La présente invention présente un dispositif médical de radiologie interventionnelle avec réalité mixte, permettant un guidage en temps réel et 3D d'une aiguille médicale (1,1A) vers au moins une cible (6) réelle dans un volume réel (3) de corps (2) d'un sujet, comprenant un volume (4) reconstruit en 3D du volume réel (3), à partir d'images médicales préalablement acquises avant l'intervention, le volume (4) reconstruit en 3D présentant des structures d'intérêt (5A, 5B, 5C) et une cible (6) reconstruite 3D correspondant à la cible (6) réelle; des moyens de stockage (7) du volume (4) reconstruit en 3D pré-enregistré avant l'intervention ; des moyens de visualisation (8) par réalité mixte pour visualiser le volume (4) reconstruit en 3D dans un référentiel virtuel de référence, destinés à être porté par un opérateur ; une aiguille médicale (1,1A) destinée à être manipulée par l'opérateur dans la partie du corps (2) jusqu'à la cible (6) réelle et des moyens (10A, 10B) de localisation et d'orientation en 3D en temps réel de l'aiguille médicale (1,1A) dans la partie du corps (2); les moyens de calcul (9) recevant les coordonnées du moyens de visualisation (8) par réalité mixte, et étant reliés aux moyens (10a, 10b) de localisation et d'orientation en 3D.

## Description

### DOMAINE DE L'INVENTION

La présente invention concerne un dispositif médical pour un usage en radiologie interventionnelle permettant de guider en temps réel, en continu et en trois dimensions l'avancée d'une aiguille dans un corps humain vers une cible, grâce à la réalité mixte.

### ETAT DE LA TECHNIQUE

Les interventions médicales guidées par l'image sont de plus en plus fréquentes par les avancées technologiques qui concernent à la fois le matériel sophistiqué et les modalités d'imagerie disponibles. Ces interventions peuvent être effectuées par voie endovasculaire ou par voie percutanée et utilisent diverses modalités d'imagerie telles que les rayons X (scanner, ou avec la tomographie dite en anglais « cone-beam computed tomography » (CBCT), fluoroscopie, angiographie soustraite), les ondes acoustiques (échographie), la résonance magnétique (imagerie par résonance magnétique (IRM)) ou encore l'imagerie multimodale (ou fusion d'images issues de différentes modalités d'images citées ci-dessus). Le but des avancées technologiques est de guider ces interventions de façon précise tout en limitant l'usage de produit de contraste iodé ou des rayons X du fait de leurs caractères néfastes pour le sujet (néphrotoxicité et effets stochastiques et déterministes sur les tissus biologiques, respectivement).

Les gestes réalisés par voie percutanée permettent de réaliser des biopsies, des ponctions, des drainages et des ablations. Les gestes percutanés sont fréquemment réalisés par guidage scanner ou CBCT lorsqu'ils concernent des structures pulmonaires, osseuses, ou digestives profondes (rétropéritonéales ou péritonéales). Les gestes réalisés en scanner présentent pour inconvénient l'usage de rayons X avec leur effet délétère connu sur les tissus biologiques et un angle limité à quelques degrés (0-15°) de la voie d'abord selon les constructeurs. Ceci limite ainsi la possibilité de réaliser certains gestes avec des axes quasi-verticaux et compliquent la possibilité de réaliser des voies d'abord avec des angles plus prononcés.

Le CBCT permet également de réaliser des gestes par voie percutanée et est restreint par un champ de vue limité par rapport au scanner et une qualité d'images moindre. Cependant, les angles des voies d'abord peuvent aller jusqu'à 45°.

L'inconvénient majeur de ces moyens de guidage (scanner et CBCT) est en effet l'usage préalable des rayons X pour guider le geste (imagerie de planification réalisée immédiatement avant le geste) mais également pour réaliser des imageries de contrôle(s) lors de l'avancée progressive de l'aiguille afin de vérifier le bon positionnement de l'aiguille. Ces imageries de contrôle accroissent davantage les temps d'intervention avec des risques de complications inhérentes, de mouvements du sujet et donc d'échec de l'intervention et d'irradiation accrue.

De nouveaux moyens de guidage ont été développés comme le guidage électromagnétique utilisant les images diagnostiques pré-interventions (ex : scanner). Ce type de procédé permet d'avancer l'aiguille en suivant simultanément le trajet de l'aiguille sur deux plans orthogonaux au sein d'un volume d'imagerie médicale issu d'une nouvelle acquisition d'imagerie de planification (ex : scanner) réalisée immédiatement avant le geste. Ceci permet de réduire voire de s'affranchir de l'usage des rayons X nécessaires lors de la progression de l'aiguille (imagerie de contrôle).

Les désavantages du système sont la nécessité d'une part de réaliser une imagerie préalable de planification irradiante par scanner et d'autre part de suivre simultanément sur un même écran (divisé en deux) l'avancée de l'aiguille selon deux plans orthogonaux. Ce suivi simultané sur les deux plans orthogonaux requiert en effet un entraînement et une attention soutenue pour reconstituer mentalement le parcours, l'orientation et la position de l'aiguille.

### INVENTION

La réalité mixte offre de nouveaux horizons en termes de guidage et s'introduit progressivement dans les salles opératoires et de radiologie interventionnelle. Celle-ci est aujourd'hui essentiellement utilisée pour la planification des interventions par la représentation en 3D d'images post-traitées issues d'imagerie médicale de type DICOM d'un sujet. Elle permet également de visualiser directement les images de fluoroscopie dynamique permettant le guidage directement dans le champ de vision de l'opérateur et non sur un écran.

La présente invention présente un dispositif médical de radiologie interventionnelle avec réalité mixte, permettant un guidage en temps réel et 3D d'une aiguille médicale vers au moins une cible réelle dans un volume réel de corps d'un sujet, comprenant :
- un volume reconstruit en 3D du volume réel, à partir d'images médicales préalablement acquises avant l'intervention,
   le volume reconstruit en 3D présentant des structures d'intérêt et une cible reconstruite 3D correspondant à la cible réelle;
- des moyens de stockage du volume reconstruit en 3D, pré-enregistré avant l'intervention ;
- un moyen de visualisation par réalité mixte pour visualiser le volume reconstruit en 3D dans un référentiel virtuel de référence, destiné à être porté par un opérateur ;
   le moyen de visualisation par réalité mixte présentant des moyens de calcul qui permettent de fusionner le volume reconstruit en 3D avec le volume réel;
   le volume reconstruit en 3D, une fois fusionné, demeurant stable dans l'espace et dans le temps sur le volume réel, la cible reconstruite 3D étant fusionnée avec la cible réelle,
- une aiguille médicale destinée à être manipulée par l'opérateur dans la partie du corps jusqu'à la cible réelle ;
- des moyens de localisation et d'orientation en 3D en temps réel de l'aiguille médicale dans la partie du corps, reliés à l'aiguille médicale, dans un référentiel réel ;
- les moyens de calcul recevant les coordonnées du moyen de visualisation par réalité mixte dans le repère réel, et étant reliés aux moyens de localisation et d'orientation en 3D de façon à recevoir le positionnement et l'orientation en 3D de l'aiguille médicale dans le référentiel réel,
   les moyens de calcul étant configurés pour :
   i) déterminer les déplacements et rotations en temps réel du moyen de visualisation dans le référentiel réel,
   ii) déterminer la position du volume réel dans le référentiel réel,
   iii) créer une aiguille virtuelle fusionnée avec l'aiguille médicale réelle en tenant compte :
      - du recalage en temps réel entre le référentiel virtuel de référence et le référentiel réel qui est fonction des déplacements et rotations en temps réel du moyen de visualisation dans le référentiel réel
      - du positionnement de l'aiguille médicale dans le référentiel réel et transmis aux moyens de calcul,
      - de la forme et de la longueur de l'aiguille médicale intégrés dans les moyens de calcul,
   afin de visualiser en temps réel via le moyen de visualisation, lors de l'intervention, le positionnement et le déplacement en 3D jusqu'à la cible reconstruite 3D de l'aiguille virtuelle dans le volume reconstruit en 3D préenregistré ;
   le positionnement virtuel et le déplacement virtuel fusionnant à travers le moyen de visualisation avec le positionnement réel et le déplacement en 3D réel de l'aiguille médicale dans le corps du sujet, pour guider le déplacement par l'opérateur de l'aiguille médicale dans le volume réel vers la cible réelle.

Le dispositif des inventeurs permet de visualiser de façon virtuelle le sujet et ses organes internes extraits d'imagerie médicale préalablement acquise et traitée, et d'y associer un objet comme une aiguille, elle-même guidée (ou localisée) en trois-dimensions et en temps réel par exemple par des ondes électromagnétiques.

L'opérateur peut ainsi guider son aiguille jusqu'à atteindre une cible dans le corps humain avec une vision directe du sujet, ses organes, la cible et l'aiguille par la réalité mixte. Le principe est de réaliser une intervention à l'aide d'une projection virtuelle des organes du sujet qui sont invisibles de l'extérieur dans le monde réel en raison de la peau, des tissus, des muscles etc.. , du corps humain, mais rendu visible par la réalité mixte.

Cette technique permettrait de s'affranchir ou du moins de réduire l'usage des rayons X et de faciliter tout type de geste percutané.

### EXPOSE DE L'INVENTION

Dans une réalisation, le dispositif médical selon l'invention présente :
- une aiguille qui doit être insérée dans le corps d'un sujet humain afin qu'elle atteigne une cible ;
- un émetteur électromagnétique fixé sur la base de l'aiguille ;
- un casque/lunette de réalité mixte qui affiche en 3D :
   1) les objets 3D (organes, tissus) obtenus par traitement des images DICOM (scanner, IRM ou CBCT) préalablement acquises et traitées du sujet et recalées avec la position réelle de celui-ci ;
   2) l'aiguille virtuelle
- un récepteur électromagnétique local posé sur la table du sujet ;
- une interface utilisateur visualisée dans les lunettes de réalité mixte qui permet de fusionner préalablement le sujet avec son propre volume post-traité et d'associer en temps réel le positionnement et l'orientation spatiale de l'aiguille par le système électromagnétique.

Le système comprend une interface qui permet de configurer les caractéristiques de l'aiguille (ou autre outils) comme sa longueur et son diamètre et son apparence virtuelle.

### DESCRIPTION DES FIGURES

D'autres objectifs, caractéristiques et avantages sortiront de la description détaillée qui suit en référence aux dessins donnés à titre illustratif et non limitatif parmi lesquels :
- les figures 1A à 1F représentent le principe de l'invention ;
- la figure 2 représente l'aiguille virtuelle avec des indications de couleur (et de longueur) ;
- la figure 3 représente le casque avec les moyens de calcul et de stockage ;
- les figures 4A à 4G représentent une illustration d'application du prototype pour une biopsie d'un nodule hépatique chez un être humain.

### DESCRIPTION GENERALE DE L'INVENTION

Le but de l'invention est de permettre à l'opérateur de guider en trois dimensions - nommé par la suite 3D- et en temps réel une aiguille médicale 1, 1A dans le corps 2 d'un sujet lors d'une séance de radiologie interventionnelle, par réalité mixte.

La réalité mixte est une projection d'objets virtuels représentant des objets réels.

La réalité mixte est une réalité augmentée.

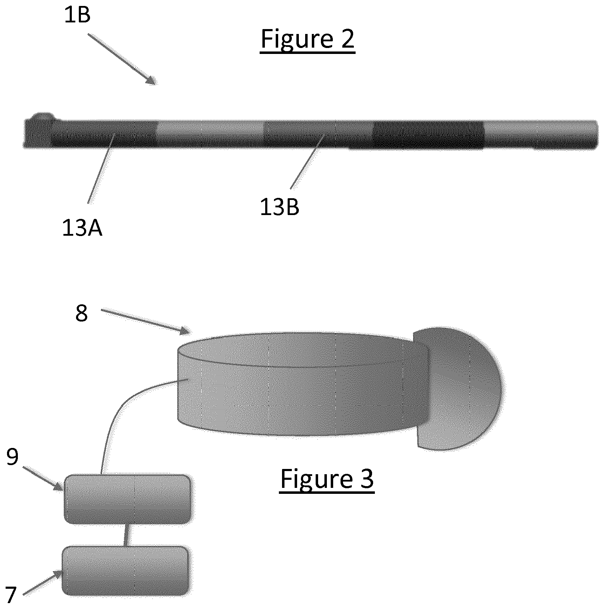

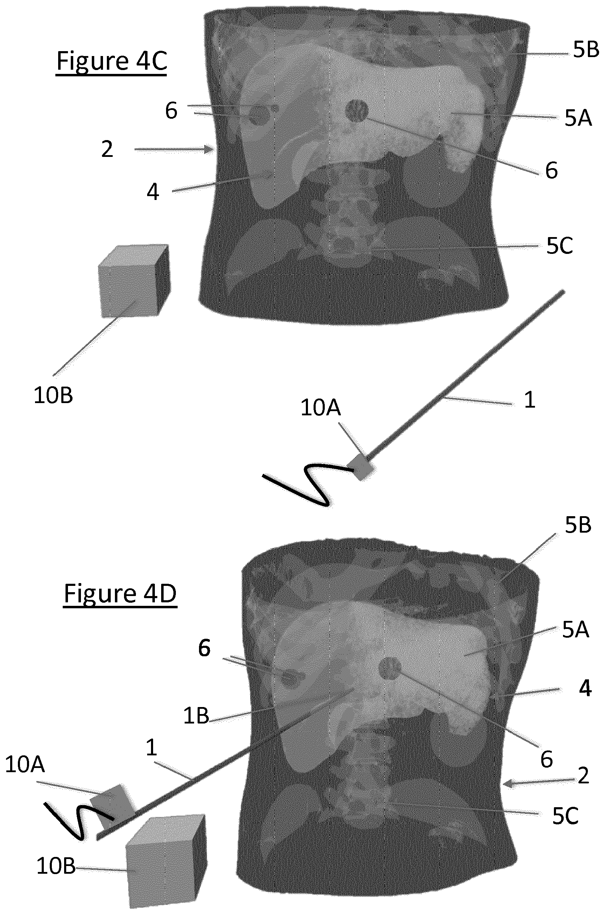

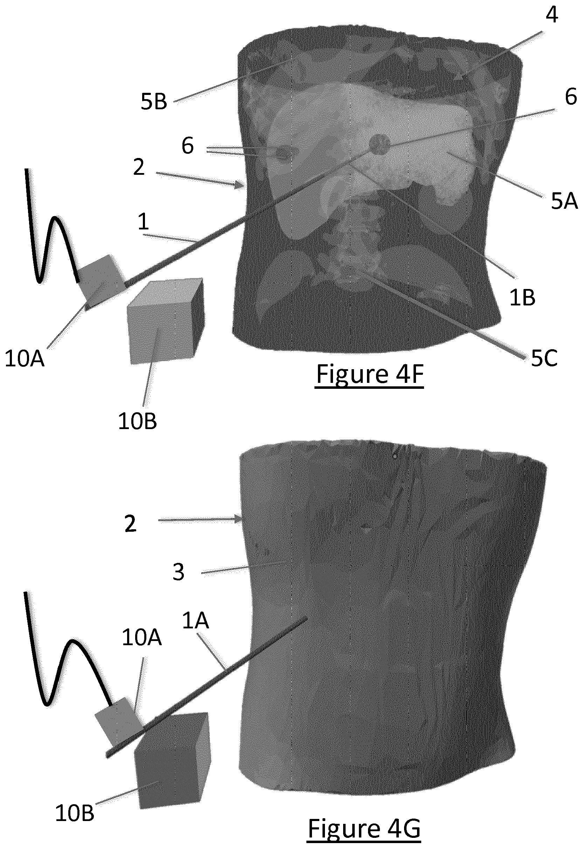

Sur les figures, par souci de simplification,
- l'aiguille médicale a été représentée par le numéro « 1 » quand elle est fusionnée avec l'aiguille virtuelle (vision avec les moyens de visualisation par réalité mixte ; figures 4C, 4D, 4F) et par le numéro « 1A » quand elle est seule (vision sans les moyens de visualisation par réalité mixte ; figure 4G) ;
- l'aiguille virtuelle a été représentée par le numéro « 1 » quand elle est fusionnée avec l'aiguille médicale (vision avec les moyens de visualisation par réalité mixte ; figures 4C, 4D, 4F) et par le numéro « 1B » quand elle est la seule visible avec le moyen de visualisation par réalité mixte (figures 2, 4D, 4F, et même si elle est demeure fusionnée dans la réalité à l'aiguille médicale qui n'est pas visible).

Le dispositif médical de radiologie interventionnelle avec réalité mixte, selon l'invention, permet un guidage en temps réel et 3D d'une aiguille médicale 1,1A vers au moins une cible 6 réelle dans un volume réel 3 de corps 2 d'un sujet.

La cible qui est représentée sur l'ensemble des figures est la cible virtuelle, mais pour des raisons de simplification, comme elle coïncide visuellement pour l'opérateur avec la cible réelle (grâce au dispositif selon l'invention), il a été choisi de mettre le même numéro 6.

Il présente :
- un volume 4 reconstruit en 3D du volume réel 3, à partir d'images médicales préalablement acquises avant l'intervention,
   le volume 4 reconstruit en 3D présentant des structures d'intérêt 5A, 5B, 5C (exemple organes) et une cible 6 reconstruite 3D correspondant à la cible 6 réelle;
- des moyens de stockage 7 du volume 4 reconstruit en 3D, pré-enregistré avant l'intervention ;
- des moyens de visualisation 8 par réalité mixte pour visualiser le volume 4 reconstruit en 3D dans un référentiel virtuel de référence, destinés à être portés par un opérateur ; les moyens de visualisation 8 par réalité mixte étant reliés à des moyens de calcul 9 qui permettent de fusionner le volume 4 reconstruit en 3D avec le volume réel 3;
   le volume 4 reconstruit en 3D, une fois fusionné, demeurant stable dans l'espace et dans le temps sur le volume réel 3, la cible 6 reconstruite 3D étant fusionnée avec la cible 6 réelle.

Les moyens de visualisation 8 par réalité mixte peuvent être par exemple un casque ou des lunettes de réalité mixte.

Le casque ou les lunettes de réalité mixte possèdent une interface logicielle qui permet de charger le volume 3D du sujet reconstruit par post traitement à partir d'images DICOM (acronyme en anglais de « Digital Imaging and COmmunications in Medicine »). Les images DICOM peuvent être issues d'images de scanner, IRM, CBCT. Les images sont post-traitées afin de construire une représentation tridimensionnelle de ce qui a été acquis. Le post traitement consiste en une phase de sélection et de segmentation des organes à reconstruire, puis une phase de reconstruction volumique à partir de ces segmentations. Enfin, une phase de mise en correspondance entre le volume 3D virtuel du sujet reconstruit et le sujet réel est nécessaire. Cette phase de fusion est appelée « recalage sujet réel - sujet virtuel ».

En d'autres termes, dans l'invention, les dimensions des objets virtuels sont identiques, par les lunettes/casque et la projection oculaire, à celles des objets réels.

Le dispositif médical présente également :
- des moyens 10a, 10b de localisation et d'orientation en 3D en temps réel de l'aiguille médicale 1,1A dans la partie du corps 2, reliés à l'aiguille médicale 1,1A, dans un référentiel réel ;
- les moyens de calcul 9 recevant les coordonnées du moyens de visualisation 8 par réalité mixte dans le repère réel, et reliés aux moyens 10a, 10b de localisation et d'orientation en 3D de façon à recevoir le positionnement et l'orientation en 3D de l'aiguille médicale 1,1A dans le référentiel réel.

Les moyens de calcul 9 sont configurés pour :
i) déterminer les déplacements et rotations en temps réel des moyens de visualisation 8 dans le référentiel réel,
ii) déterminer la position du volume réel 3 dans le référentiel réel,
iii) créer une aiguille virtuelle 1,1B fusionnée avec l'aiguille médicale 1,1A réelle en tenant compte :
   - du recalage en temps réel entre le référentiel virtuel de référence et le référentiel réel qui est fonction des déplacements et rotations en temps réel des moyens de visualisation 8 dans le référentiel réel
   - du positionnement de l'aiguille médicale 1,1A dans le référentiel réel et transmis aux moyens de calcul 9,
   - de la forme et de la longueur de l'aiguille médicale 1,1A intégrés dans les moyens de calcul 9.

Les moyens de calcul 9 permettent de visualiser en temps réel via les moyens de visualisation 8, lors de l'intervention, le positionnement et le déplacement en 3D jusqu'à la cible 6 reconstruite 3D de l'aiguille virtuelle 1,1B dans le volume 4 reconstruit en 3D préenregistré ;
le positionnement virtuel et le déplacement virtuel fusionnant à travers les moyens de visualisation 8 avec le positionnement réel et le déplacement en 3D réel de l'aiguille médicale 1,1A dans le corps 2 du sujet, pour guider le déplacement par l'opérateur de l'aiguille médicale 1,1A dans le volume réel 3 vers la cible 6 réelle.

Avantageusement, les moyens de calcul 9 déterminent automatiquement le recalage en temps réel entre le référentiel virtuel de référence et le référentiel réel,
les moyens de calcul 9 ayant été configurés pour créer:
- un premier objet virtuel à sélectionner dans le référentiel virtuel de référence et dont les coordonnées sont acquises ;
- un second objet virtuel à sélectionner dans le référentiel virtuel de référence et dont les coordonnées sont acquises,
afin de calculer le décalage entre les deux référentiels,
et donc faire fusionner en permanence et en temps réel aiguille réelle 1, 1A et aiguille virtuelle 1,1B lors du recalage automatique.

Le dispositif médical peut présenter des moyens permettant de calculer le décalage entre les référentiels virtuel et réel, tels que :
- une imagerie per-intervention (scanner ou CBCT) ;
- des QR codes métalliques disposés sur le sujet, destinés à être imagés en même temps que le sujet avec les moyens d'imagerie et reconstruits dans le référentiel virtuel,
- la surface de peau du sujet reconstruite dans le volume 4 reconstruit en 3D ; exemple il peut être pris la surface de peau du pubis, sternum, nombril, épaules, etc..

Les moyens de calcul 9 peuvent être configurés pour réaliser un second repère virtuel fixe dans le référentiel virtuel de référence à proximité de l'aiguille virtuelle 1,1B pour quantifier le déplacement de l'aiguille virtuelle 1,1B.

Dans une première réalisation, les moyens 10a, 10b de localisation et d'orientation en 3D présentent :
- au moins un capteur de position 10a émettant des ondes électromagnétiques fixé sur l'aiguille médicale 1,1A ;
- un dispositif de réception 10b des ondes électromagnétiques.
Il est à tout fait possible de mettre le récepteur d'ondes électromagnétiques sur l'aiguille et l'émetteur d'ondes électromagnétiques en dehors de l'aiguille sur un endroit de la table

Dans une deuxième réalisation, les moyens 10a, 10b de localisation et d'orientation en 3D sont :
- des moyens optiques tels que des caméras, et/ou
- des dispositifs mécaniques tels qu'un dispositif haptique.

Avantageusement, l'aiguille médicale 1,1A est apte à être utilisée lors de biopsies, ponctions, drainages et ablations pour tout type de cibles du corps 2 humain (exemples : cutanées, mammaires, osseuses, thoraciques, abdominales ou vasculaires artériels et veineux).

Les images médicales utilisées sont de type DICOM (ex : scanner, IRM et CBCT) et sont traitées (ex : segmentation, seuillage, modélisation en volume 3D).

Avantageusement, le volume 4 reconstruit en 3D présente une partie reconstruite comme la surface du sujet (exemple : la peau), des organes d'intérêt et la ou les cible (s).

Le dispositif médical peut comporter des seconds moyens 10A, 10B de localisation et d'orientation en 3D en temps réel, de tout ou partie du sujet pour déterminer sa position dans le référentiel réel, et les calculs reliés à ces seconds moyens sont configurés pour recalculer en continu les mouvements de l'aiguille afin d'assurer un recalage en continu entre les référentiels virtuel et réel.

Avantageusement, l'aiguille virtuelle 1,1B peut présenter des repères visibles comme une échelle avec des segments 13A, 13B (exemple : code couleur) permettant d'indiquer la longueur d'aiguille virtuelle 1,1B.

Dans une autre réalisation, l'aiguille virtuelle 1,1B peut présenter uniquement un contour qui coïncide avec le contour de l'aiguille réelle dans la réalité mixte.

### DESCRIPTION DETAILLEE D'UN MODE DE REALISATION DE L'INVENTION

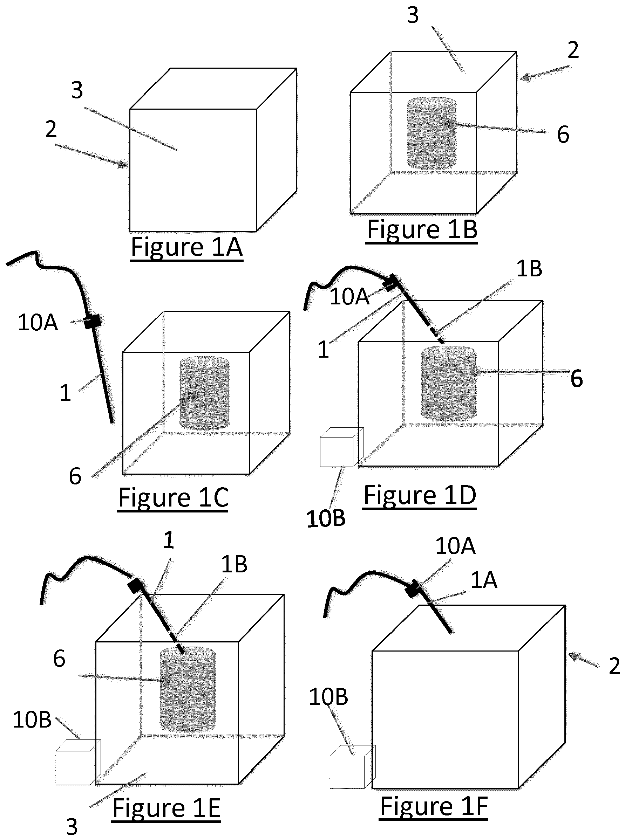

La Figure 1 A représente la vision réelle du sujet de l'invention (ici représenté par un cube), et la figure 1 B représente une vue mixte du sujet et de sa cible au travers de lunettes/casque de réalité mixte. Il est ainsi possible de voir au travers le sujet et d'y apercevoir la cible et sa localisation (i.e. il est possible de voir à travers la peau et les organes).
La figure 1C représente une vue mixte du sujet, de sa cible ainsi que de l'aiguille sur laquelle est fixée l'émetteur électromagnétique, lui-même étant relié au récepteur électromagnétique.
La figure 1D représente une vue mixte du sujet et de sa cible ainsi que de l'aiguille qui progresse dans le sujet pour atteinte la cible. Le mouvement de l'aiguille est visualisé avec son pendant virtuel en 3D et en temps réel.
La figure 1E représente une vue mixte du sujet et de sa cible ainsi que de l'aiguille qui a atteint la cible.
La figure 1F représente la vision réelle du sujet et de l'aiguille introduite et ayant atteint sa cible (sans moyen de visualisation 8 de réalité mixte).

Pendant l'intervention, l'opérateur utilise une aiguille. Sur la base de cette aiguille est fixé un émetteur d'ondes électromagnétiques qui permet de connaître son positionnement et son orientation dans l'espace. Cette aiguille, dont les caractéristiques (longueur, diamètre) ont été prédéfinies par le biais de l'interface utilisateur du casque/lunettes de réalité mixte, nécessite d'être préalablement fusionnée avec son pendant virtuel. Cette phase de fusion est appelée « recalage aiguille virtuelle 1,1B - aiguille réelle 1, 1A ».

Le système de localisation peut utiliser toute sorte de méthode de localisation (ondes électromagnétiques, optiques, mécanique).

Dans le cas d'une localisation électromagnétique, un boitier récepteur d'ondes électromagnétiques est fixé sur la table, proche de la cible.

L'aiguille peut être insérée à n'importe quelle position et suivre un quelconque angle selon le choix de l'opérateur.

La précision maximale de position de l'aiguille est obtenue quand l'émetteur est situé proche du champ d'intervention.

L'opérateur avance progressivement l'aiguille grâce au guidage par réalité mixte de la position de l'aiguille jusqu'à atteindre sa cible (Figure 1D). La position et l'orientation de l'aiguille sont obtenues en temps réel par le système de localisation électromagnétique. A partir de ces informations, l'aiguille virtuelle 1,1B (préalablement fusionnée avec l'aiguille réelle) est visible entièrement et en permanence grâce à la réalité mixte, même si l'aiguille réelle n'est plus complètement visible puisque dans le corps 2 du sujet.

Les mains de l'opérateur sont ainsi libres, et son regard est augmenté et orienté vers le sujet et sa cible. Le volume reconstruit est fusionné et calé de façon statique sur le corps 2 du sujet. L'ensemble du corps 2 du sujet et sa projection augmentée de ses organes sont ainsi fusionnés et visibles sur son champ de vision lors de l'avancée de l'aiguille et l'opérateur n'a pas besoin d'écran pour se guider.

Création de l'aiguille virtuelle 1,1B illustrée sur la figure 2:
L'aiguille virtuelle 1,1B est un objet virtuel permettant de représenter virtuellement l'aiguille réelle afin de pouvoir suivre le déplacement de l'aiguille virtuelle 1,1B dans le volume 3D. L'aiguille virtuelle 1,1B est composée de 2 éléments :
- La base, qui représente la position du capteur 10A par rapport à l'émetteur 10B dans l'espace du casque ;
- Le corps multicolore représente le corps de l'aiguille, chaque couleur représente par exemple 5cm sur le corps réel (la taille et l'apparence est configurable par l'interface). Le contrôle de l'aiguille se fait via le récepteur du capteur de position 3D.
Comme indiqué précédemment, la base de l'aiguille est l'endroit où se situe le capteur 10a, il doit être dans la bonne position pour que le réel corresponde au virtuel, c'est le rôle de l'étape de « recalage aiguille virtuelle 1,1B - aiguille réelle » vu précédemment. La sélection d'un modèle à charger se fait via une interface graphique affichée en réalité mixte. Le modèle 3D, une fois chargé est fusionné avec le corps 2 du sujet dans le monde réel via un processus de calibration manuelle obtenue grâce à la superposition du modèle 3D et du corps 2 du sujet. Ce processus de calibration est appelé « recalage sujet virtuel - sujet réel». Il est réalisé à l'aide de l'interface graphique qui affiche des boutons virtuels mais il peut aussi être réalisé à l'aide d'une manette de jeux connectée au casque ou à l'aide d'un clavier sans fil connecté au casque.

L'objectif est d'étalonner maintenant les repères du casque virtuel, du monde réel et des aiguilles réelle et virtuelle.

Dans un premier temps, il est nécessaire de faire coïncider les repères virtuels du casque et ceux de l'aiguille réelle et virtuelle.

Le recalage « aiguille virtuelle 1,1B - aiguille réelle » peut être réalisé de deux façons.

Soit manuellement à l'aide de l'interface graphique (ou d'une manette de jeux ou d'un clavier sans fil connecté au casque).

Soit semi-automatiquement : on fait apparaître des cubes virtuels à des positions 3D connues par le système. L'utilisateur doit alors amener la base de l'aiguille virtuelle 1,1B (pour le moment décalée de l'aiguille réelle puisque c'est le but de cette étape de les fusionner) tour à tour sur chacun de ces cubes. Un algorithme calcul ensuite automatiquement le décalage existant afin de le compenser et ainsi faire coïncider l'aiguille virtuelle 1,1B et l'aiguille réelle.

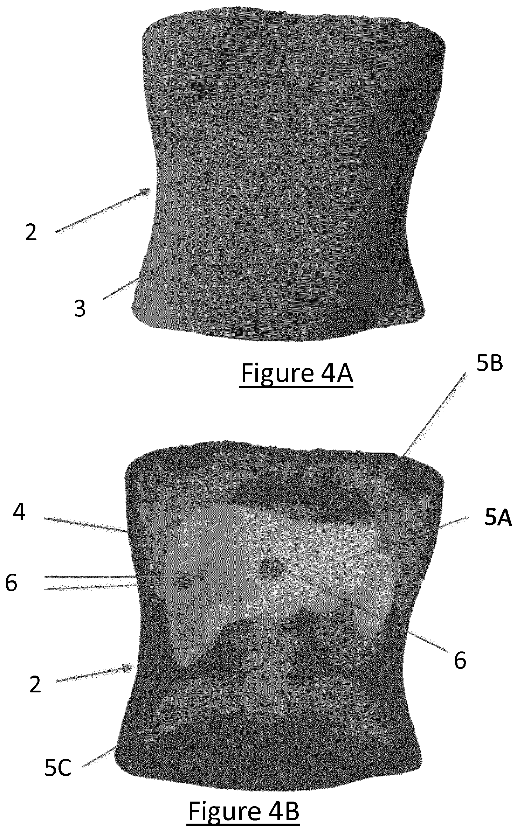

Il a été représenté sur les figures 4 une application du dispositif médical selon l'invention.

La figure 4A représente la vue externe réelle de l'abdomen du sujet.

Les figures 4B à 4E représentent la vue par l'opérateur (médecin ou autre..) porteur des lunettes/casque de réalité mixte de l'abdomen du sujet, qui voit le volume reconstruit en 3D projeté et fusionné avec et sur le volume réel.

La figure 4B représente la vue de l'abdomen du sujet et de son volume reconstruit (visualisation du foie 5A, de trois nodules hépatiques 6 dont un nodule cible, des côtes 5B et du rachis 5C obtenue après traitement d'images DICOM de son scanner abdominal) par réalité mixte au travers de lunettes/casque.

La figure 4C représente la vue en réalité mixte du sujet (réel et virtuel) avec l'aiguille et son pendant virtuel, l'émetteur et le récepteur.

La figure 4D représente la progression de l'aiguille 1 vers la cible avec un trajet ascendant, antéro-postérieur, sous costal (pour éviter la plèvre). L'aiguille 1 est à présent dans le foie 5A et est dirigée par l'opérateur en temps réel et en 3D jusqu'à sa cible.

La figure 4E représente l'aiguille 1 qui a atteint sa cible grâce au dispositif ;

La figure 4F représente la vision réelle (sans casque/lunettes) du sujet et de l'aiguille médicale 1A.

## Revendications

**1.** Dispositif médical de radiologie interventionnelle avec réalité mixte, permettant un guidage en temps réel et en 3D d'une aiguille médicale (1,1A) vers au moins une cible (6) réelle dans un volume réel (3) de corps (2) d'un sujet, comprenant :
- un volume (4) reconstruit en 3D du volume réel (3), à partir d'images médicales préalablement acquises avant l'intervention,
le volume (4) reconstruit en 3D présentant des structures d'intérêt (5A, 5B, 5C) et une cible (6) reconstruite 3D correspondant à la cible (6) réelle;
- des moyens de stockage (7) du volume (4) reconstruit en 3D, pré-enregistré avant l'intervention ;
- des moyens de visualisation (8) par réalité mixte pour visualiser le volume (4) reconstruit en 3D dans un référentiel virtuel de référence, destinés à être portés par un opérateur ;
les moyens de visualisation (8) par réalité mixte étant reliés à des moyens de calcul (9) qui permettent de fusionner le volume (4) reconstruit en 3D avec le volume réel (3);
le volume (4) reconstruit en 3D, une fois fusionné, demeurant stable dans l'espace et dans le temps sur le volume réel (3), la cible (6) reconstruite 3D étant fusionnée avec la cible (6) réelle,
- une aiguille médicale (1,1A) destinée à être manipulée par l'opérateur dans la partie du corps (2) jusqu'à la cible (6) réelle ;
- des moyens (10A, 10B) de localisation et d'orientation en 3D, en temps réel, de l'aiguille médicale (1,1A) dans la partie du corps (2), reliés à l'aiguille médicale (1,1A), dans un référentiel réel ;
- les moyens de calcul (9) recevant les coordonnées des moyens de visualisation (8) dans le repère réel, et étant reliés aux moyens (10A, 10B) de localisation et d'orientation en 3D, de façon à recevoir le positionnement et l'orientation en 3D de l'aiguille médicale (1,1A) dans le référentiel réel,
les moyens de calcul (9) étant configurés pour :
i) déterminer les déplacements et rotations en temps réel des moyens de visualisation (8) dans le référentiel réel,
ii) déterminer la position du volume réel (3) dans le référentiel réel,
iii) créer une aiguille virtuelle (1,1B) fusionnée avec l'aiguille médicale (1,1A) réelle en tenant compte :
• du recalage en temps réel entre le référentiel virtuel de référence et le référentiel réel qui est fonction des déplacements et rotations en temps réel des moyens de visualisation (8) dans le référentiel réel,
• du positionnement de l'aiguille médicale (1,1A) dans le référentiel réel et transmis aux moyens de calcul (9),
• de la forme et de la longueur de l'aiguille médicale (1,1A) intégrés dans les moyens de calcul (9),
afin de visualiser en temps réel à travers les moyens de visualisation (8), lors de l'intervention, le positionnement et le déplacement en 3D jusqu'à la cible (6) reconstruite 3D de l'aiguille virtuelle (1,1B) dans le volume (4) reconstruit en 3D préenregistré ;
le positionnement virtuel et le déplacement virtuel fusionnant à travers le moyens de visualisation (8) avec le positionnement réel et le déplacement en 3D réel de l'aiguille médicale (1,1A) dans le corps (2) du sujet, pour guider le déplacement par l'opérateur de l'aiguille médicale (1,1A) dans le volume réel (3) vers la cible (6) réelle.

**2.** Dispositif médical selon la revendication 1, dans lequel, les moyens de calcul (9) déterminent automatiquement le recalage en temps réel entre le référentiel virtuel de référence et le référentiel réel,
les moyens de calcul (9) ayant été configurés pour créer:
- un premier objet virtuel à sélectionner dans le référentiel virtuel de référence et dont les coordonnées sont acquises ;
- un second objet virtuel à sélectionner dans le référentiel virtuel de référence et dont les coordonnées sont acquises,
afin de calculer le décalage entre les deux référentiels,
et donc faire fusionner en temps réel aiguille médicale (1,1A) et aiguille virtuelle (1,1B) lors du recalage automatique.

**3.** Dispositif médical selon l'une des revendications précédentes, dans lequel le dispositif médical comprend des moyens permettant de calculer le décalage entre les référentiels virtuel et réel, tels que :
- une imagerie per-intervention (scanner ou CBCT) ;
- des QR codes métalliques disposés sur le sujet, destinés à être imagés en même temps que le sujet avec les moyens d'imagerie et reconstruits dans le référentiel virtuel,
- la surface de peau du sujet reconstruite dans le volume (4) reconstruit en 3D.

**4.** Dispositif médical selon l'une des revendications précédentes, dans lequel les moyens de calcul (9) sont configurés pour réaliser un second repère virtuel fixe dans le référentiel virtuel de référence à proximité de l'aiguille virtuelle (1,1B) pour quantifier le déplacement de l'aiguille virtuelle (1,1B).

**5.** Dispositif médical selon l'une des revendications précédentes, dans lequel les moyens de calcul (9) sont configurés pour visualiser par le casque/lunettes 3D la trajectoire et l'avancée 3D de l'aiguille virtuelle (1,1B) jusqu'à la cible (6) reconstruite 3D dans le volume (4) reconstruit en 3D.

**6.** Dispositif médical selon l'une des revendications précédentes, dans lequel les moyens (10a, 10b) de localisation et d'orientation en 3D présentent :
- au moins un capteur de position (10A) émettant des ondes électromagnétiques situé sur l'aiguille médicale (1,1A) ;
- un dispositif de réception (10B) des ondes électromagnétiques.

**7.** Dispositif médical selon l'une des revendications 1 à 5, dans lequel les moyens (10a, 10b) de localisation et d'orientation en 3D sont :
- des moyens optiques tels que des caméras, et/ou
- des dispositifs mécaniques tels qu'un dispositif haptique.

**8.** Dispositif médical selon l'une des revendications précédentes, dans lequel l'aiguille médicale (1,1A) est apte à être utilisée lors de biopsies, ponctions, drainages et ablations pour tout type de cibles (6) du corps (2).

**9.** Dispositif médical selon l'une des revendications précédentes, dans lequel les images médicales utilisées sont de type DICOM et sont traitées par segmentation, seuillage, conversion en volume 3D par la technique de rendu de volume.

**10.** Dispositif médical selon l'une des revendications précédentes, dans lequel le volume (4) reconstruit en 3D présente une partie reconstruite comme la surface du sujet, des structures d'intérêt (5A, 5B, 5C) et la ou les cibles (6).

**11.** Dispositif médical selon l'une des revendications précédentes, dans lequel le dispositif médical comporte des secondes moyens (10a, 10b) de localisation et d'orientation en 3D en temps réel, de tout ou partie du sujet pour déterminer sa position dans le référentiel réel, et les calculs reliés à ces seconds moyens sont configurés pour recalculer en continu les mouvements de l'aiguille afin d'assurer un recalage en continu entre les référentiels virtuel et réel.

**12.** Dispositif médical selon l'une des revendications précédentes, dans lequel l'aiguille virtuelle (1,1B) présente des repères visibles permettant d'indiquer la longueur d'aiguille virtuelle (1,1B).

**14.** Dispositif médical selon l'une des revendications précédentes, dans lequel l'aiguille virtuelle (1,1B) présente uniquement un contour destiné à coïncider avec le contour de l'aiguille réelle (1, 1A) dans la réalité mixte.

**15.** Dispositif médical selon l'une des revendications précédentes, dans lequel le volume réel (3) de corps (2) du sujet est une partie du corps (2) du sujet.

**16.** Dispositif médical selon l'une des revendications précédentes, dans lequel le volume réel (3) de corps (2) du sujet est l'ensemble du corps (2) du sujet.
